# EUROPEAN PATENT SPECIFICATION

(11) **EP 1 780 189 B1**
(45) Date of publication and mention of the grant of the patent: **07.05.2008**
(21) Application number: 05022779.2
(22) Date of filing: 19.10.2005
(51) Int. Cl.: C07C 2/30, C08F 110/02, C07C 11/00

(54) **Oligomerization of ethylene**
Oligomerisierung von Ethylene
Oligomérisation d'ethylène

(43) Date of publication of application: 02.05.2007
(73) Proprietor: Saudi Basic Industries Corporation, Riyadh 11422 (SA); Linde AG, 80807 München (DE)
(72) Inventor: Schneider, Richard, 82449 Uffing (DE); Fritz, Peter M., 82008 Unterhaching (DE); Muschelknantz, Sebastian, 81479 München (DE); Bölt, Heinz, 82515 Wolfratshausen (DE); Ali, Talal, 11551 Riyadh (SA); Mousa, Fuad, 11551 Riyadh (SA)
(74) Representative: Winkler, Andreas Fritz Ernst

(56) References cited:
- DE-C1- 4 338 414
- US-A- 2 320 251

## Description

The present invention relates to a method for oligomerisation of ethylene to form linear alpha-olefins in an oligomerisation reactor in the presence of solvent, catalyst and co-catalyst, wherein a reaction product containing linear alpha-olefins or alpha-olefins separated from the reaction product are passed to a heat exchanger for cooling thereof.

Oligomerisation methods for preparing linear alpha-olfins are widely known in the art. This methods are easily carried out in the presence of a catalyst preferably comprising a zirconium component, such as zirconium tetraisobutyrate, and an aluminum component as activator, such as ethyl aluminum sesquichloride.

Usually, a reaction product discharged from the oligomerisation reactor may comprise solvent, catalyst, co-catalyst and liquid linear alpha-olefins. This reaction product is further processed, for example the catalyst and co-catalyst may be deactivated and removed from the alpha-olefins. Additionally, the reaction product may be passed to a heat exchanger for cooling thereof.

In the prior art, it is desirable to adjust a wall temperature of that heat exchanger to be as low as possible, as the lower the wall temperature the smaller the heat exchanger may be designed. However, it has been found that high molecular weight oligomers obtained in that reaction product (for example linear alpha-olefins having more than 20 carbon atoms) have a high tendency for plugging in heat exchangers on surfaces thereof having relatively low temperature. This may result in a reduced heat transfer efficiency in that heat exchanger.

DE 43 38 414 C1 discloses a process for preparing linear alpha-olefins by oligomerisation of ethylene in the presence of an organic solvent and a homogenous liquid catalyst consisting of a zirconium compound and an organo metallic compound, wherein the oligomerisation is carried out at a pressure of 31 to 50 bar in a reactor, into which bottom ethylene is introduced and from which lower part the oligomerisation product is removed together with solvent and catalyst.

US 2,320,251 discloses a process for converting hydrocarbon gases into liquid hydrocarbons at elevated temperatures at which the reaction products remain in the vapor phase. The reaction products are reduced by indirect heat exchange with charging gases to a temperature close to but not below the dew point thereof and then suddenly the temperature of the partially cooled reaction products is reduced below the dew point by direct contact with cooler liquid of such nature that a large portion thereof remains liquid at the temperature to which the reaction products are reduced.

It is therefore an object of the present invention to provide a method for oligomerisation of ethylene which overcomes the drawbacks of the prior art. Especially, a method shall be provided wherein plugging of equipment for carrying out the oligomerisation, especially, plugging of heat exchangers, may be avoided.

This object is achieved in that the wall temperature of the heat exchanger is adjusted to be higher than the pour points of the linear alpha-olefins obtained.

Preferably, wherein the reaction product comprises C₄-C₂₀₊ olefins.

In one embodiment, the wall temperature is higher than 35°C, preferably higher than 40°C, more preferably higher than 70°C.

Also preferred, adjustment of the wall temperature is achieved by selection of an appropriate coolant, air recirculation in an air cooler, air heating in an air cooler, or combinations thereof.

The method is preferably characterized in that the catalyst comprises a zirconium salt of organic acids, preferably having the formula ZrCl₄₋ₘXₘ, wherein X = OCOR or OSO₃R' with R and R' being independently alkyl, alkene or phenyl, and wherein 0 < m < 4.

Finally, the co-catalyst may be at least one organoaluminum compound, preferably Al(C₂H₅)₃, Al₂Cl₃(C₂H₅)₃ or AlCl(C₂H₅)₂.

Surprisingly, it was found that plugging of reaction equipment, especially of the heat exchanger, may be avoided if the wall temperature of that heat exchanger is adjusted to be higher than the pour points of the linear alpha-olefins to be cooled. Additionally, the thermal efficiency of the heat exchanger may be maintained. If the method is run in the heat exchanger at a temperature higher than the pour points of the linear alpha-olefins, the linear alpha-olefins can not solidify as deposits on the surfaces of the heat exchanger.

Preferably, the wall temperature of the heat exchanger may be adjusted by selection of appropriate coolant, air recirculation in an air cooler, air heating in an air cooler, or combinations thereof. In air coolers, external air circulation in a closed section of the air cooler may be installed. A heating bundle may be installed, which can be electrically heated, or heated by means of steam, hot oil or warm water. In addition, louvers may be installed at the air inlet and / or outlet. The tube wall temperature may be kept at the envisaged level by means of a controller. In shell-and-tube heat exchangers the applicable cooling medium may be optimized, e.g. cooling water may be taken from the cold water supply header or from the warm return header. In addition a cooling water recirculation may be installed to adjust tube wall temperature.

## Claims

1. Method for oligomerisation of ethylene to form linear alpha-olefins in an oligomerisation reactor in the presence of solvent, catalyst and co-catalyst, wherein a reaction product containing linear alpha-olefins or alpha-olefins separated from the reaction product are passed to a heat exchanger for cooling thereof, **characterized in that** the wall temperature of the heat exchanger is adjusted to be higher than the pour points of the linear alpha-olefins obtained.

2. Method according to claim 1, wherein the reaction product comprises C₄-C₂₀₊ olefins.

3. Method according to claim 1 or 2, wherein the wall temperature is higher than 35°C, preferably higher than 40°C, more preferably higher than 70°C.

4. Method according to any of the preceding claims, wherein adjustment of the wall temperature is achieved by selection of an appropriate coolant, air recirculation in an air cooler, air heating in an air cooler, or combinations thereof.

5. Method according to any of the preceding claims, wherein the catalyst comprises a zirconium salt of organic acids, preferably having the formula ZrCl₄₋ₘX_{m,} wherein X = OCOR or OSO₃R' with R and R' being independently alkyl, alkene or phenyl, and wherein 0 < m < 4.

6. Method according to any of the preceding claims, wherein the co-catalyst is at least one organoaluminum compound, preferably Al(C₂H₅)₃, Al₂Cl₃(C₂H₅)₃ or AlCl(C₂H₅)₂.

## Patentansprüche

1. Verfahren zur Oligomerisation von Ethylen, um lineare alpha-Olefine in einem Oligomerisationsreaktor in der Gegenwart von Lösungsmittel, Katalysator und Co-Katalysator zu bilden, wobei ein Reaktionsprodukt, das lineare alpha-Olefine enthält, oder von dem Reaktionsprodukt abgetrennte alpha-Olefine zu einem Wärmeaustauscher zum Abkühlen geführt wird bzw. werden, **dadurch gekennzeichnet, dass** die Wandtemperatur des Wärmeaustauschers höher als die Fließpunkte der erhaltenen linearen alpha-Olefine eingestellt wird.

2. Verfahren nach Anspruch 1, wobei das Reaktionsprodukt C₄-C₂₀₊-Olefine umfasst.

3. Verfahren nach Anspruch 1 oder 2, wobei die Wandtemperatur höher als 35°C, bevorzugt höher als 40°C, bevorzugter höher als 70°C ist.

4. Verfahren nach einem der vorangehenden Ansprüche, wobei die Einstellung der Wandtemperatur durch Auswahl eines geeigneten Kühlmittels, Luftrezirkulation in einem Luftkühler, Lufterwärmen in einem Luftkühler oder Kombinationen derselben erreicht wird.

5. Verfahren nach einem der vorangehenden Ansprüche, wobei der Katalysator ein Zirkoniumsalz organischer Säuren umfasst, bevorzugt mit der Formel zrCl₄₋ₘXₘ, wobei X = OCOR oder OSO₃R' ist, wobei R und R' unabhängig Alkyl, Alken oder Phenyl sind, und wobei 0 < m < 4 ist.

6. Verfahren nach einem der vorangehenden Ansprüche, wobei der Co-Katalysator wenigstens eine Organoaluminiumverbindung ist, bevorzugt Al(C₂H₅)₃, Al₂Cl₃(C₂H₅)₃ oder AlCl(C₂H₅)₂.

## Revendications

1. Procédé d'oligomérisation d'éthylène pour former des alpha-oléfines linéaires dans un réacteur d'oligomérisation en présence de solvant, de catalyseur et de co-catalyseur, où un produit réactionnel contenant des alpha-oléfines linéaires ou des alpha-oléfines séparées du produit réactionnel passe(nt) dans un échangeur thermique pour y être refroidi(es), **caractérisé en ce que** la température de la paroi de l'échangeur thermique est ajustée pour être supérieure aux points de figeage des alpha-oléfines linéaires obtenues.

2. Procédé selon la revendication 1, dans lequel le produit réactionnel comprend des oléfines en C₄-C₂₀₊.

3. Procédé selon la revendication 1 ou 2, dans lequel la température de la paroi est supérieure à 35 °C, de préférence supérieure à 40 °C, plus préférablement supérieure à 70 °C.

4. Procédé selon l'une quelconque des revendications précédentes, dans lequel l'ajustement de la température de la paroi est obtenu en choisissant un caloporteur approprié, une recirculation d'air dans un refroidisseur d'air, un chauffage d'air dans un refroidisseur d'air, ou des combinaisons de ceux-ci.

5. Procédé selon l'une quelconque des revendications précédentes, dans lequel le catalyseur comprend un sel de zirconium d'acides organiques, ayant de préférence la formule ZrCl₄₋ₘXₘ, où X = OCOR ou OSO₃R', R et R' étant indépendamment un groupe alkyle, alcène ou phényle, et où 0 < m < 4.

6. Procédé selon l'une quelconque des revendications précédentes, dans lequel le co-catalyseur est au moins un composé d'organoaluminium, de préférence Al(C₂H₅)₃, Al₂Cl₃(C₂H₅)₃ ou AlCl(C₂H₅)₂.
